# EUROPÄISCHE PATENTSCHRIFT

(11) **EP 1 239 777 B1**
(45) Veröffentlichungstag und Bekanntmachung des Hinweises auf die Patenterteilung: **08.10.2003**
(21) Anmeldenummer: 01980220.6
(22) Anmeldetag: 27.07.2001
(51) Int. Cl.: A61B 17/00, A61B 18/14

(54) **MEDIZINISCHES INSTRUMENT MIT ABNEHMBAREM WERKZEUG**
MEDICAL INSTRUMENT COMPRISING A DETACHABLE TOOL
INSTRUMENT MEDICAL COMPORTANT UN OUTIL AMOVIBLE

(30) Priorität: 03.08.2000 DE 10038576
(43) Veröffentlichungstag der Anmeldung: 18.09.2002
(73) Patentinhaber: Karl Storz GmbH & Co. KG, 78532 Tuttlingen (DE)
(72) Erfinder: SCHNEIDER, Martin, D-78588 Denkingen (DE); BACHER, Uwe, D-78532 Tuttlingen (DE)
(74) Vertreter: Heuckeroth, Volker, Dr.
(86) Internationale Anmeldenummer: EP0108696
(87) Internationale Veröffentlichungsnummer: WO02011620

(56) Entgegenhaltungen:
- EP-A- 0 738 501
- DE-A- 19 854 313
- DE-C- 19 815 228
- DE-U- 9 317 535
- US-A- 5 571 137

## Beschreibung

Die Erfindung betrifft ein medizinisches Instrument, mit einem Schaft, und mit einem an einem distalen Ende des Schafts abnehmbar befestigten Werkzeug, wobei der Schaft einen Innenschaft und einen den Innenschaft umgebenden und relativ zu diesem längs verschiebbaren Rohrschaft aufweist, wobei das Werkzeug in einer Verriegelungsstellung des Rohrschafts am Schaft verriegelt und in einer Freigabestellung des Rohrschafts vom Schaft abnehmbar ist.

Ein derartiges Instrument ist aus der DE-C-198 15 228 bekannt.

Ein Instrument der eingangs genannten Art wird für chirurgische Eingriffe am menschlichen oder tierischen Körper, insbesondere im Rahmen der minimal-invasiven Chirurgie, verwendet.

Je nach Funktion und Ausgestaltung des am distalen Ende des Schafts angeordnete Werkzeuges kann das Instrument ein Schneid-, Faß- und/oder Koagulationsinstrument sein, um einige Beispiele zu nennen. Im Falle eines Schneidinstruments kann das Werkzeug als Klinge oder in Form von zwei Maulteilen ausgebildet sein, die in der Art von Scherenteilen schneidend zusammenwirken. Im Falle eines Koagulationsinstruments kann das Werkzeug als Elektrode ausgebildet sein, die vom proximalen Ende des Instruments her mit Hochfrequenzstrom beaufschlagbar ist. Die vorliegende Erfindung läßt sich bei allen zuvor genannten Instrumententypen und auch bei anderen Instrumententypen, wie Nadelhaltern, einsetzen.

Während bei herkömmlichen Instrumenten das Werkzeug mit dem distalen Ende des Schafts fest verbunden war und sich allenfalls bei einer vollständigen Zerlegung des Instruments zu Reinigungszwecken vom Schaft abnehmen ließ, ist aus der zuvor genannten DE-C-198 15 228 ein Instrument bekannt, bei dem das Werkzeug am distalen Ende vom Schaft abnehmbar ist, ohne daß dazu das Instrument zerlegt werden muß.

Bei diesem bekannten Instrument ist das Werkzeug eine Elektrodenspitze zum Schneiden und Koagulieren von Geweben mittels Hochfrequenzstrom. Die Elektrodenspitze ist am Schaft auswechselbar gehalten, wobei der Schaft einen Innenschaft und einen den Innenschaft umgebenden Rohrschaft aufweist, der relativ zum Innenschaft längsverschiebbar ist. Die Elektrodenspitze ist an dem Innenschaft gehalten, wobei der Innenschaft radial nach außen bewegbare Fixierabschnitte aufweist, die den in den Innenschaft eingesteckten Halter der Elektrodenspitze in ihrer nach außen bewegten Freigabestellung freigeben, in einer nach innen bewegten Verriegelungsstellung dagegen in axialer Richtung festlegen. Der Rohrschaft überdeckt die Fixierabschnitte in einer vorgeschobenen Verriegelungsstellung und gibt die Fixierabschnitte in einer zurückgezogenen Freigabestellung frei.

Ein vergleichbares Instrument mit einem abnehmbaren Werkzeug am distalen Ende des Schafts ist aus der DE-C-43 41 735 bekannt, wobei dieses bekannte Instrument als Werkzeug zwei relativ zueinander bewegliche Maulteile aufweist, die über ein Kraftübertragungselement mittels einer Handhabe am proximalen Ende des Schafts betätigbar sind. Das Kraftübertragungselement erstreckt sich dabei durch den Innenschaft hindurch. Auch eine Ausgestaltung des eingangs genannten medizinischen Instruments mit einem in dem Innenschaft längs bewegbaren Kraftübertragungselement zum Bewegen des Werkzeugs ist im Rahmen der vorliegenden Erfindung möglich.

Der Vorteil eines medizinischen Instruments mit einem abnehmbaren Werkzeug besteht darin, daß verschiedene Werkzeuge während einer mit dem Instrument durchgeführten Operation gegeneinander ausgetauscht werden können. Auf diese Weise müssen nicht mehrere vollständige Instrumente, sondern nur verschiedene Werkzeuge bereitgehalten werden, die dann wahlweise gegeneinander ausgetauscht werden können. Im Fall, daß das Werkzeug eine Elektrode ist, können bspw. verschiedene Werkzeuge mit verschiedenen Elektrodenformen verwendet werden. Ein solches Instrument ist somit gegenüber herkömmlichen Instrumenten universell verwendbar.

Um eine Akzeptanz eines medizinischen Instruments, bei dem das Werkzeug vom Schaft abnehmbar und gegen andere Werkzeuge austauschbar ist, gegenüber herkömmlichen Instrumenten, die keine abnehmbaren Werkzeuge besitzen, zu erreichen, ist es erforderlich, daß die Handhabung des Instruments zum Wechseln des Werkzeuges einfach ist und der Werkzeugwechsel schnell und bequem durchführbar ist.

Bei dem aus der zuvor genannten DE-C-198 15 228 bekannten Instrument ist zur Vereinfachung der Handhabbarkeit vorgeschlagen worden, den Rohrschaft in der vorgeschobenen Verriegelungsstellung um die Längsachse des Innenschafts in eine Position verdrehbar auszugestalten, in der es durch eine bajonettartige Verriegelung am Innenschaft in Längsrichtung festgelegt ist. Zum Abnehmen des Werkzeugs vom distalen Ende des Schafts muß der Rohrschaft von Hand zurückgeschoben werden, indem zwei Griffteile, von denen eines mit dem Rohrschaft verbunden ist, so um die Längsachse des Innenschafts gegeneinander verdreht werden, daß ein Stift der bajonettartigen Verriegelung in eine Längsnut des Bajonetts eingreift, wonach der Rohrschaft von Hand zurückgezogen werden kann.

An diesem Betätigungsmechanismus des bekannten Instruments ist nachteilig, daß der Rohrschaft von Hand relativ zum Innenschaft verdreht und zurückgezogen werden muß. Diese Bauweise des Betätigungsmechanismus erfordert somit eine Zwei-Hand-Bedienung, eine Einhand-Bedienung ist zumindest erschwert.

Der Erfindung liegt daher die Aufgabe zugrunde, ein medizinisches Instrument der eingangs genannten Art dahingehend weiterzubilden, daß die Handhabung zum Abnehmen bzw. Anbringen des Werkzeugs am distalen Ende des Schafts weiter vereinfacht wird.

Erfindungsgemäß wird diese Aufgabe hinsichtlich des eingangs genannten medizinischen Instruments dadurch gelöst, daß ein im wesentlichen quer zur Längsrichtung des Rohrschafts bewegliches Betätigungselement vorgesehen ist, das mit dem Rohrschaft derart in Wirkverbindung steht, daß durch eine Bewegung des Betätigungselements von einer ersten Stellung in eine zweite Stellung der Rohrschaft aus der Verriegelungsstellung in die Freigabestellung bewegt wird und umgekehrt.

Bei dem erfindungsgemäßen Instrument ist demnach ein Betätigungsmechanismus vorgesehen, der ein im wesentlichen quer zur Längsrichtung des Rohrschafts bewegliches Betätigungselement aufweist. Dieses bewegliche Betätigungselement steht mit dem Rohrschaft derart in Wirkverbindung, daß die Bewegung des beweglichen Betätigungselements in eine Bewegung des Rohrschafts in dessen Längsrichtung umgesetzt wird. Der Vorteil des im wesentlichen quer zur Längsrichtung des Rohrschafts beweglichen Betätigungselements besteht darin, daß sich ein solches Betätigungselement, wenn es am Handgriff des Instruments angeordnet ist, bspw. mittels eines Druckknopfes durch Daumendruck einfach bedienen läßt. Eine derartige Betätigung des Instruments zum Verschieben des Rohrschafts aus der Verriegelungsstellung in die Freigabestellung gewährleistet eine Einhandbedienung des Instruments zum Auswechseln des Werkzeuges. Das Verschieben des Rohrschaftes relativ zum Innenschaft kann somit mit derselben Hand erfolgen, die auch das Instrument als solches hält, während mit der anderen Hand bereits gleichzeitig das abzunehmende Werkzeug oder das anzubringende Werkzeug gehandhabt werden kann.

In einer bevorzugten Ausgestaltung weist das Betätigungselement einen Eingriffsabschnitt auf, der mit einer Ausnehmung am Rohrschaft relativ zu dieser beweglich in Eingriff steht, wobei der Eingriffsabschnitt und die Ausnehmung eine solche Formgebung aufweisen, daß eine im.wesentlichen quer zur Längsrichtung gerichtete Bewegung des Betätigungselements in eine Längsbewegung des Rohrschafts umgesetzt wird.

Diese Maßnahme führt zu dem weiteren Vorteil einer konstruktiv besonders einfachen Bauweise des Betätigungsmechanismus. Gleichzeitig wird vorteilhafterweise eine Verdrehsicherung für den Schaft geschaffen. Denkbar ist jedoch auch, die Wirkverbindung zwischen dem Betätigungselement und dem Rohrschaft über ein Getriebe mit Kegelrädern zu realisieren.

Dabei ist es bevorzugt, wenn der Eingriffsabschnitt und die Ausnehmung schräg zur Längsrichtung des Schafts und schräg zur Bewegungsrichtung des Betätigungselements verlaufen.

Durch diese Maßnahme wird ein linearer Betätigungsmechanismus geschaffen, d.h. der Bewegungsweg des Rohrschafts ist proportional zum Bewegungsweg des Betätigungselements. Diese Ausgestaltung erweist sich als vorteilhaft, weil sich der Rohrschaft gleichmäßig mit der Bewegung des Betätigungselements verschiebt.

In einer weiteren bevorzugten Ausgestaltung ist das Betätigungselement von der zweiten Stellung in eine dritte Stellung bewegbar, in der das Betätigungselement mit der Ausnehmung vollständig außer Eingriff kommt, wobei der Rohrschaft in der dritten Stellung vom Innenschaft abnehmbar bzw. auf diesem montierbar ist.

Diese Maßnahme hat den Vorteil, daß mittels des Betätigungselements nicht nur der Rohrschaft zum Wechseln des Werkzeuges verschoben werden kann, sondern das Betätigungselement gleichzeitig die Funktion einer Endarretierung für den Rohrschaft besitzt, um den Rohrschaft vom Innenschaft bzw. vom Handgriff zu lösen.

In einer weiteren bevorzugten Ausgestaltung ist das Betätigungselement in die erste Stellung vorgespannt.

Diese Maßnahme hat zum einen den Vorteil, daß der Rohrschaft aufgrund der Vorspannung des Betätigungselements in die erste Stellung aus der Freigabestellung selbsttätig in die Verriegelungsstellung zurückkehrt, wenn das Betätigungselement losgelassen wird. Zum anderen wird durch diese Maßnahme der weitere Vorteil erreicht, daß der Rohrschaft in der Verriegelungsstellung relativ zum Innenschaft durch die Vorspannung des Betätigungselements in der Verriegelungsstellung festgelegt ist.

In einer weiteren bevorzugten Ausgestaltung ist das Betätigungselement von der ersten Stellung in die zweite Stellung gegen eine erste Kraft bewegbar und zum Abnehmen des Rohrschafts vom Innenschaft von der zweiten Stellung in die dritte Stellung gegen eine zweite Kraft bewegbar, die größer ist als die erste Kraft.

Hierbei ist von Vorteil, daß ein zweistufiger Betätigungsmechanismus geschaffen wird, dessen erste Stufe dem Verschieben des Rohrschafts relativ zum Innenschaft dient, um das Werkzeug am distalen Ende des Schafts auszuwechseln, während die zweite Stufe dem Zerlegen des Instruments, d.h. zum Abnehmen des Rohrschafts dient. Darüber hinaus kann die das Instrument bedienende Person aufgrund der unterschiedlichen Widerstände feststellen, wann das Ende der ersten Bewegungsstufe erreicht ist, nämlich wenn sich beim weiteren Bewegen des Betätigungselements der Widerstand stufenartig erhöht. Auf diese Weise kann die Person bei einem Werkzeugwechsel vermeiden, daß sie das Betätigungselement bis in die dritte Stellung durchdrückt, in der der Rohrschaft vom Instrument abnehmbar ist.

In diesem Zusammenhang ist bevorzugt, wenn das Betätigungselement zur Montage des Rohrschafts auf dem Innenschaft gegen die erste Kraft allein in die dritte Stellung bewegbar ist.

Im Zusammenhang mit der zuvor genannten Ausgestaltung hat diese Maßnahme den Vorteil, daß nur zum Abnehmen des Rohrschafts vom Innenschaft das Betätigungselement von der zweiten in die dritte Stellung gegen eine höhere Kraft bewegt werden muß, wodurch in jenem Fall vermieden wird, daß beim Werkzeugwechsel das Betätigungselement in die dritte Stellung, d.h. in die Lösestellung zum Abnehmen des Rohrschafts, bewegt wird. Da bei der Montage des Rohrschafts von dem Innenschaft dem zuvor genannten Sicherheitsaspekt nicht Rechnung getragen werden muß, wird durch die zuvor genannte Maßnahme auch die Handhabung des erfindungsgemäßen Instruments bei der Montage des Rohrschafts auf dem Innenschaft vereinfacht, weil nur die erste Kraft überwunden werden muß, um den Rohrschaft auf dem Innenschaft zu montieren.

In einer weiteren bevorzugten Ausgestaltung weist das Betätigungselement zwei Eingriffsabschnitte auf, die an gegenüberliegenden Umfangspositionen des Rohrschafts in entsprechende Ausnehmungen eingreifen.

Durch diese Maßnahme wird der Betätigungsmechanismus noch weiter hinsichtlich seiner Funktionssicherheit verbessert, weil das Betätigungselement mit den zwei Eingriffsabschnitten an gegenüberliegenden Umfangspositionen symmetrisch am Rohrschaft angreift, wodurch ein Verkanten oder Verklemmen des Betätigungselements mit dem Schaft vermieden wird.

In einer weiteren bevorzugten Ausgestaltung weist das Betätigungselement eine sich an den Eingriffsabschnitt oder die Eingriffsabschnitte anschließende Öffnung auf, deren lichter Durchmesser größer als der Außendurchmesser des Rohrschafts ist.

Diese Maßnahme ist insbesondere in Verbindung mit der vorhergehenden Maßnahme von Vorteil, wonach das Betätigungselement zwei Eingriffsabschnitte aufweist, so daß das Betätigungselement dann insgesamt einen Durchgang in Form eines Schlüsselloches aufweisen kann. In der dritten Stellung des Betätigungselements umgibt die Öffnung den Rohrschaft konzentrisch, so daß der Rohrschaft dann mit seinem proximalen Abschnitt durch die öffnung aus dem Betätigungselement herausgezogen werden kann.

In einer weiteren bevorzugten Ausgestaltung ist das Betätigungselement gegen eine erste Feder abgestützt.

Diese Maßnahme hat den Vorteil einer konstruktiv einfachen Bauweise, um das Betätigungselement in die erste Stellung vorzuspannen, wobei das Betätigungselement gegen die Kraft der ersten Feder von der ersten in die zweite Stellung bewegbar ist.

Dabei ist es weiterhin bevorzugt, wenn das Betätigungselement in einem Führungselement geführt ist, das mit einem Bedienelement, bspw. in Form eines Druckknopfes, verbunden ist, derart, daß durch Drücken des Bedienelements das Führungselement das Betätigungselement mitnimmt.

Diese Maßnahme ist insbesondere dann vorteilhaft, wenn gemäß einer weiteren bevorzugten Ausgestaltung das Betätigungselement relativ zu dem Führungselement verschiebbar ist.

Diese Ausgestaltung hat den Vorteil, daß sich das Betätigungselement vom Bedienungselement entkoppelt unabhängig von dem Bedienelement bewegen läßt. Dies ist insbesondere für eine erleichterte Montage des Rohrschafts von Vorteil, um gemäß einer zuvor genannten Ausgestaltung das Betätigungselement gegen die erste geringere Kraft allein in die dritte Stellung zu bewegen, wenn nämlich die zweite Gegenkraft am Führungselement angreift.

In einer weiteren bevorzugten Ausgestaltung ist das Betätigungselement von der zweiten Stellung in die dritte Stellung direkt oder indirekt zusätzlich gegen eine zweite Feder abgestützt bewegbar.

Das Vorsehen einer zweiten Feder zusätzlich zur ersten Feder stellt eine konstruktiv vorteilhaft einfache Bauweise dar, damit das Betätigungselement von der ersten Stellung in die zweite Stellung gegen eine erste Kraft und von der zweiten in die dritte Stellung nur gegen eine höhere zweite Kraft bewegt werden kann.

In einer weiteren bevorzugten Ausgestaltung stützt sich die zweite Feder gegen das Führungselement ab und wird erst bei der Bewegung des Betätigungselements von der zweiten Stellung in die dritte Stellung zum Abnehmen des Rohrschafts wirksam.

Mit dieser Maßnahme wird auf konstruktiv einfache Weise der zuvor beschriebene zweistufige Betätigungsmechanismus für das Betätigungselement geschaffen.

Dabei ist es weiterhin bevorzugt, wenn der lichte Innendurchmesser der zweiten Feder größer ist als der Außenumfang des Betätigungselements, wobei die erste Feder innerhalb der zweiten Feder angeordnet ist.

Durch diese Maßnahme wird der Vorteil erzielt, daß sich das Betätigungselement zum Montieren des Rohrschafts am Instrument aufgrund der zuvor genannten Ausgestaltung auch allein gegen die Kraft der ersten Feder in die dritte Stellung bewegen läßt, wodurch die Montage des Rohrschafts vereinfacht ist.

In einer weiteren bevorzugten Ausgestaltung ist die zweite Feder härter als die erste Feder.

Wenn die zweite Feder härter ist als die erste Feder, läßt sich der Übergang des Betätigungselements von der ersten Bewegungsstufe in die zweite Bewegungsstufe für den Benutzer des Instruments noch leichter erkennen, weil auf diese Weise eine Art Anschlag für das Ende der ersten Bewegungsstufe geschaffen wird, indem ab der zweiten Stellung die zweite härtere Feder wirksam wird.

In einer weiteren bevorzugten Ausgestaltung ist am Betätigungselement distalseitig eine Anlaufschräge für das proximale Ende des Rohrschafts ausgebildet, so daß das Betätigungselement beim Auflaufen des proximalen Endes auf die Anlaufschräge in die dritte Stellung bewegt wird.

Diese Maßnahme hat den Vorteil, daß sich der Rohrschaft ohne Betätigung des Bedienelementes auf dem Innenschaft montieren läßt, indem der Rohrschaft in besonders einfach zu bedienender Weise lediglich auf den Innenschaft aufgeschoben wird. Das proximale Ende des Rohrschafts bewegt dabei das Betätigungselement insbesondere in Verbindung mit der zuvor genannten Ausgestaltung lediglich gegen die erste geringere Kraft allein bis in die dritte Stellung. Der Rohrschaft wird dann noch soweit nach proximal geschoben, bis die Ausnehmung am Rohrschaft auf Höhe des Eingriffsabschnitts des Betätigungselements zu liegen kommt, wobei in dieser Stellung das Betätigungselement bei entsprechender Federvorspannung, wie zuvor erwähnt, selbsttätig in die erste Stellung springt und dabei der Eingriffsabschnitt in die Ausnehmung am Rohrschaft eingreift. Auf diese Weise wird ein vorteilhaft einfach zu bedienender Rastmechanismus zum Montieren des Rohrschafts auf dem Innenschaft geschaffen.

In einer weiteren bevorzugten Ausgestaltung ist das Betätigungselement an einem Handgriff des Instruments bedienbar angeordnet.

Auf diese Weise läßt sich das Betätigungselement in Verbindung mit einem Bedienelement, wie einem Druckknopf, besonders handhabungsfreundlich zum Wechseln des Werkzeuges oder zum Abnehmen des Rohrschafts mit derselben Hand bedienen, die auch den Handgriff greift.

In einer weiteren bevorzugten Ausgestaltung weist ein distales Ende des Innenschafts eine bajonettartige Ausnehmung mit einem sich zunächst axial und anschließend umfänglich erstreckenden Abschnitt auf, wobei am proximalen Ende des Werkzeugs ein Halter ausgebildet ist, der mit einem sich quererstreckenden Abschnitt in die bajonettartige Ausnehmung einsetzbar ist, und wobei am Rohrschaft zumindest eine axiale Ausnehmung ausgebildet ist, in die der sich quer erstreckende Abschnitt des Halters in der Verriegelungsstellung des Rohrschafts eingreift.

Während das aus der DE-C-198 15 228 bekannte Instrument eine spannzangenartige Halterung für das Werkzeug mit radial beweglichen Teilen aufweist, hat diese Ausgestaltung den Vorteil, daß auf bewegliche Teile verzichtet werden kann, die den Nachteil haben, bruchgefährdet zu sein, zumal wenn diese wie bei dem bekannten Instrument als elastische Zungen ausgebildet sind.

Weiterhin ist es bevorzugt, wenn der Rohrschaft in der Verriegelungsstellung relativ zum Innenschaft vorgeschoben und in der Freigabestellung zurückgezogen ist, weil dann insbesondere der zuvor erwähnte Bajonettverschluß konstruktiv einfach ausgestaltet sein kann.

Bei einer bevorzugten Ausgestaltung des erfindungsgemäßen Instruments ist das Werkzeug eine Elektrode zum Koagulieren oder Schneiden von Gewebe.

Während dies eine bevorzugte Ausgestaltung des erfindungsgemäßen Instruments darstellt, ist die Erfindung jedoch nicht auf ein solches HF-Instrument beschränkt, sondern läßt sich auch bei anderen Instrumenten vorteilhaft einsetzen.

Weitere Merkmale und Vorteile ergeben sich aus der nachfolgenden Beschreibung und den beigefügten Zeichnungen.

Ein Ausführungsbeispiel der Erfindung ist in den Zeichnungen dargestellt und wird in der nachfolgenden Beschreibung näher erläutert. Es zeigen:
- Fig. 1: eine perspektivische Gesamtansicht eines medizinischen Instruments mit abgenommenem Werkzeug und abgenommenem Rohrschaft;
- Fig. 2: das Instrument in Fig. 1 in Seitenansicht in zusammengesetztem Zustand, teilweise mit Aufbrechungen, wobei ein Detail in einem Teilbild vergrößert dargestellt ist;
- Fig. 3: das Instrument in Fig. 1 und 2 in einer Betriebsstellung, in der das Werkzeug abnehmbar bzw. anbringbar ist, wobei ein Detail in einem Teilbild vergrößert dargestellt ist;
- Fig. 4: das Instrument in Fig. 1 bis 3 in einer Betriebsstellung, in der der Rohrschaft abnehmbar bzw. anbringbar ist, wobei ein Detail in einem Teilbild vergrößert dargestellt ist;
- Fig. 5: ein Betätigungselement des Instruments in Fig. 1 bis 4 im Längsschnitt in Seitenansicht im vergrößerten Maßstab;
- Fig. 6: das Betätigungselement in Fig. 5 in einer Vorderansicht;
- Fig. 7: ein Führungselement des Instruments in Fig. 1 bis 4 in einer Seitenansicht im vergrößerten Maßstab;
- Fig. 8: das Führungselement in Fig. 7 im Längsschnitt in Vorderansicht; und
- Fig. 9a) bis c): eine schematische Darstellung des Vorgangs des Montierens des Rohrschafts am Instrument in drei Teilbildern, die das Instrument ausschnittsweise im Bereich des Betätigungselements zeigen.

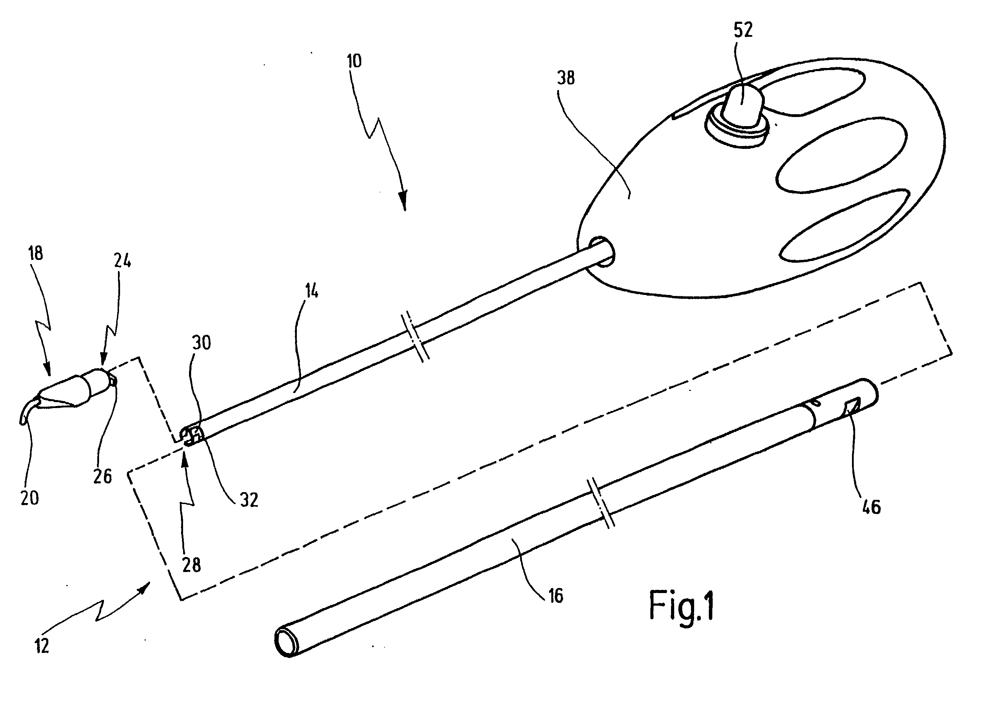

In Fig. 1 bis 4 ist ein mit dem allgemeinen Bezugszeichen 10 versehenes medizinisches Instrument dargestellt.

Das Instrument 10 ist ein Instrument zum Koagulieren bzw. Schneiden von Gewebe mittels Hochfrequenzstrom.

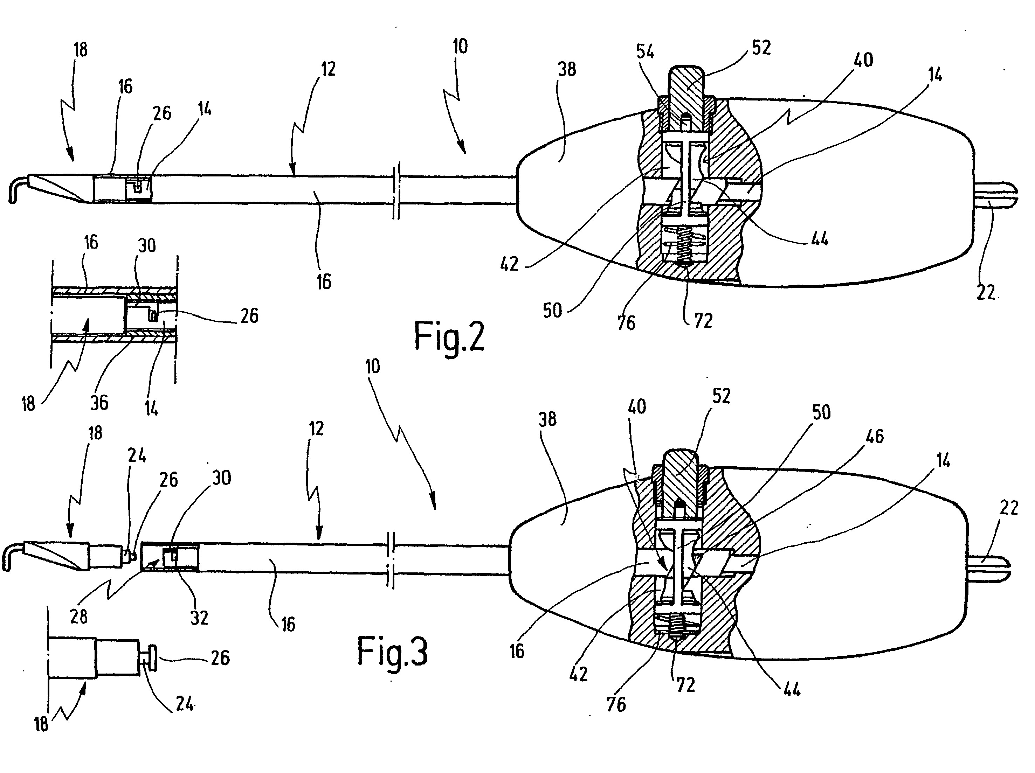

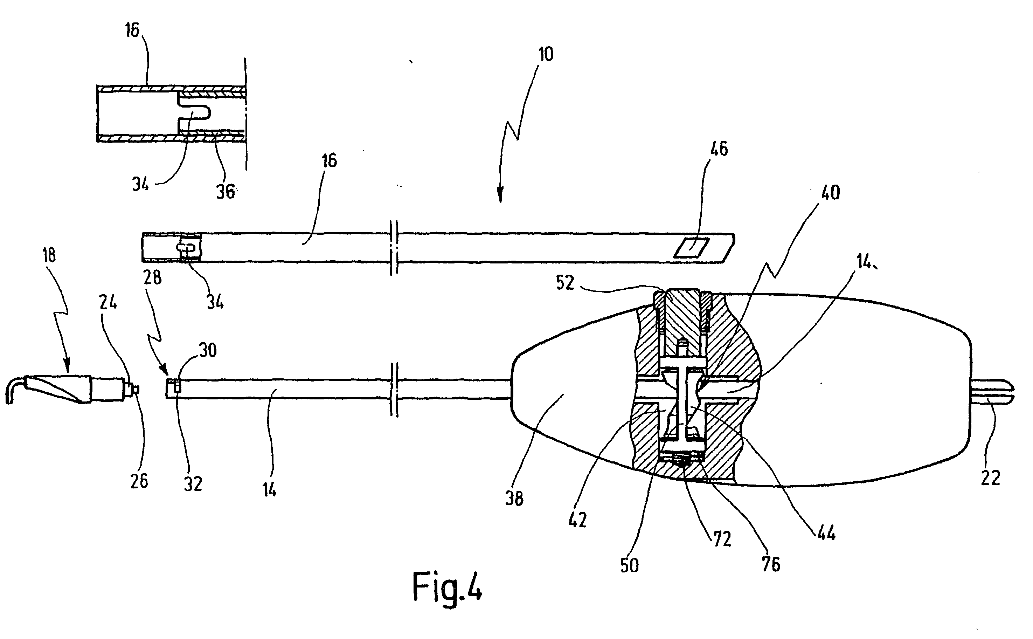

In Fig. 1 ist das Instrument 10 perspektivisch in seine Hauptbaugruppen zerlegt dargestellt. Das Instrument 10 weist einen Schaft 12 auf, der einen Innenschaft 14 und einen den Innenschaft 14 umgebenden Rohrschaft 16 aufweist. In Fig. 1 und 4 ist der Rohrschaft 16 im von dem Innenschaft 14 abgenommenen Zustand dargestellt. In Fig. 2 und 3 ist der Rohrschaft 16 auf dem Innenschaft 14 montiert.

Am distalen Ende des Schafts 12 ist ein Werkzeug 18 abnehmbar befestigt. In Fig. 1, 3 und 4 ist das Werkzeug 18 im abgenommenen und in Fig. 2 im an dem Schaft 12 befestigten Zustand dargestellt.

Das Werkzeug 18 weist entsprechend der Ausgestaltung des Instruments 10 als Instrument zum Koagulieren und Schneiden von Gewebe mittels Hochfrequenzstrom eine Elektrodenspitze 20 auf.

Der Innenschaft 14 dient als Stromzuführung für die Elektrodenspitze 20, wobei sich der Innenschaft 14 bis zum proximalen Ende des Instruments 10 erstreckt und dort elektrisch leitend mit einem Kontakt 22 verbunden ist, an den ein mit einer nicht dargestellten Hochfrequenzstromquelle verbundener nicht dargestellter HF-Stecker anschließbar ist.

Zum abnehmbaren Befestigen des Werkzeugs 18 an dem Schaft 12 weist das Werkzeug 18 an seinem proximalen Ende einen Halter 24 auf, der in Fig. 3 in einem vergrößerten Ausschnitt des proximalen Endes des Werkzeugs 18 in Draufsicht dargestellt ist. Der Halter 24 weist an seinem äußersten proximalen Ende einen sich quer erstreckenden Abschnitt 26 auf, der in Form eines T ausgebildet ist.

Das distale Ende des Innenschafts 14 weist eine bajonettartige Ausnehmung 28 auf, die einen sich vom äußersten distalen Ende des Innenschafts 14 aus nach proximal erstreckenden axialen Abschnitt 30 und einen sich daran anschließenden teilumfänglich erstreckenden Abschnitt 32 aufweist. Genauer gesagt weist die bajonettartige Ausnehmung am distalen Ende des Innenschafts 14 zwei sich gegenüberliegende axial erstreckende Abschnitte 30 und zwei sich diametral gegenüberliegende umfängliche Abschnitte 32 auf.

Der Halter 24 des Werkzeugs 18 läßt sich mit seinem sich quer erstreckenden Abschnitt 26 in die bajonettartige Ausnehmung 28, d.h. zunächst in die sich axial erstreckenden Abschnitte 30 und durch Drehen um seine Längsachse um etwa 90° im Uhrzeigersinn (von distal gesehen) in die sich umfänglich erstreckenden Abschnitte 32 der bajonettartigen Ausnehmung 28 einsetzen.

Um das Werkzeug 18 am Schaft 12 festzulegen, weist der Rohrschaft 16 im Bereich seines distalen Endes eine axiale, als Langloch ausgebildete Ausnehmung 34 auf, in die der sich quer erstreckende Abschnitt 26 des Halters 24 des Werkzeugs 18 eingreift. Die Ausnehmung 34 ist dabei in einer Hülse 36 vorgesehen, die in dem Rohrschaft 16 im Bereich seines distalen Endes angeordnet ist. Durch das Vorsehen der Hülse 36 innerhalb des Rohrschafts 16 kann der Rohrschaft 16 eine durchgehend geschlossene Außenwand aufweisen. Der sich quer erstreckende Abschnitt 26 des Halters 24 weist eine Querabmessung auf, die etwa dem Innendurchmesser des distalen Endes des Rohrschafts 16 distalseitig der Hülse 36 und etwa dem Außendurchmesser der Hülse 36 entspricht.

Der Rohrschaft 16 ist relativ zum Innenschaft 14 längsverschiebbar, wobei das Werkzeug 18 in einer relativ zum Innenschaft 14 vorgeschobenen Verriegelungsstellung des Rohrschafts 16, die in Fig. 2 dargestellt ist, am Schaft verriegelt ist, indem der sich quer erstreckende Abschnitt 26 des Halters 24 in dieser Verriegelungsstellung des Rohrschafts 16 in dessen Ausnehmung 34 eingreift.

In einer in Fig. 3 dargestellten, relativ zum Innenschaft 14 zurückgezogenen Freigabestellung des Rohrschafts 16 ist das Werkzeug 18 dagegen vom Schaft 12 abnehmbar, weil dann der sich quer erstreckende Abschnitt 26 des Halters 24 des Werkzeugs 18 nicht mehr in die Ausnehmung 34 des Rohrschafts 16 eingreift und sich somit aus der bajonettartigen Ausnehmung 28 des Innenschafts 14 zunächst durch Drehen um 90° entgegen dem Uhrzeigersinn und schließlich durch Herausziehen aus dem sich axial erstreckenden Abschnitt 30 der bajonettartigen Ausnehmung 28 abnehmen läßt.

Um den Rohrschaft 16 aus der in Fig. 2 dargestellten vorgeschobenen Verriegelungsstellung in die in Fig. 3 dargestellte zurückgezogene Freigabestellung zu bewegen, weist das Instrument 10 einen Betätigungsmechanismus auf, der in einem Handgriff 38 des Instruments 10 am proximalen Ende des Schafts 12 integriert ist.

Dieser Betätigungsmechanismus weist ein Betätigungselement 40 auf, das in einer sich quer zur Längsrichtung des Schafts 12 erstreckenden Sacklochbohrung 42 des Handgriffs 38 angeordnet ist.

Das Betätigungselement 40 ist im wesentlichen quer zur Längsrichtung des Schafts 12 und damit zur Längsrichtung des Rohrschafts 16 beweglich in der Sacklochbohrung 42 aufgenommen, wobei das Betätigungselement 40 mit dem Rohrschaft 16 derart in Wirkverbindung steht, daß der Rohrschaft 16 aus der Verriegelungsstellung durch eine Bewegung des Betätigungselements 40 von einer ersten, in Fig. 2 dargestellten Stellung in eine in Fig. 3 dargestellte zweite Stellung in die Freigabestellung und umgekehrt bewegt wird.

Die Bewegungsrichtung des Betätigungselements 40 ist in dem gezeigten Ausführungsbeispiel senkrecht zur Längsrichtung des Schafts 12.

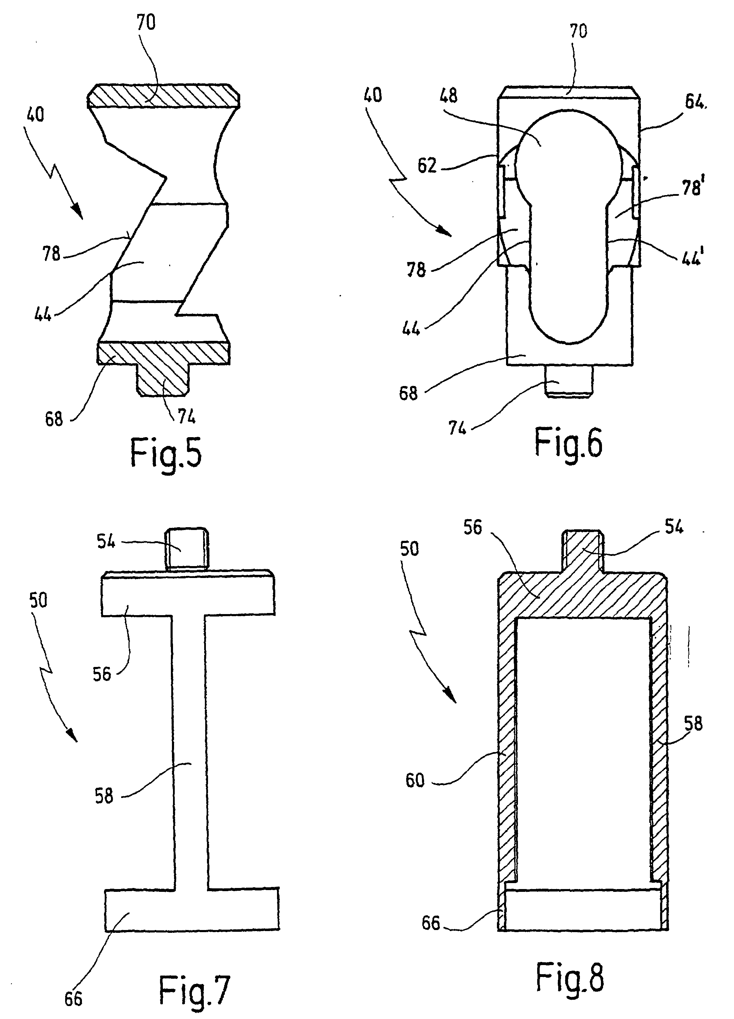

Dazu weist das Betätigungselement, das in Fig. 5 und 6 im vergrößerten Maßstab in Alleinstellung dargestellt ist, einen Eingriffsabschnitt 44 auf, der mit einer Ausnehmung 46 am Rohrschaft 16 relativ zur Ausnehmung 46 beweglich in Eingriff steht. Der Eingriffsabschnitt 44 und die Ausnehmung 46 weisen dabei eine solche Formgebung auf, daß eine im wesentlichen quer zur Längsrichtung des Schafts 12 gerichtete Bewegung des Betätigungselements 40 in eine Längsbewegung des Rohrschafts 16 umgesetzt wird.

Diese zuvor erwähnte Umsetzung der Bewegung des Betätigungselements 40 im wesentlichen quer zur Längsrichtung des Schafts 12 in eine Längsbewegung des Rohrschafts 16 wird dabei dadurch erreicht, daß der Eingriffsabschnitt 44 und die Ausnehmung 46 am Rohrschaft 16 schräg zur Längsrichtung des Schafts 12 und schräg zur Bewegungsrichtung des Betätigungselements 40 verlaufen.

Das Betätigungselement 40 weist dabei insgesamt zwei Eingriffsabschnitte 44 und 44' auf, die an gegenüberliegenden Umfangspositionen des Rohrschafts 16 in entsprechende Ausnehmungen 46 des Rohrschafts eingreifen. In Fig. 6 sind beide Eingriffsabschnitte 44 und 44' dargestellt. Die Ausnehmungen 46 im Bereich des proximalen Endes des Rohrschafts 16 sind durch entsprechende Ausbrüche gebildet, in denen der Umfang des Rohrschafts 16 vermindert ist. Entsprechend ragen die Eingriffsabschnitte 44 und 44' radial soweit nach innen, daß sie in die beiden Ausnehmungen 46 beidseitig des Rohrschafts 16 eingreifen können.

Das Betätigungselement 40 weist gemäß Fig. 6 eine sich an die beiden Eingriffsabschnitte 44 und 44' anschließende Öffnung 48 auf, deren lichter Durchmesser etwas größer als der Außendurchmesser des Rohrschafts 16 ist. Insgesamt weist der Körper des Betätigungselements 40 einen Durchgang in Form eines Schlüssellochs auf, wie aus Fig. 6 ersichtlich ist. Der als Langloch ausgebildete Abschnitt dieses Schlüssellochs wird durch die Eingriffsabschnitte 44 und 44' berandet, während die Öffnung 48 den sich daran anschließenden kreisrunden Abschnitt des schlüssellochartigen Durchganges bildet. Der lichte Durchmesser des Langlochabschnitts ist in Querrichtung demnach kleiner als der Außendurchmesser des Rohrschafts 16 im Bereich außerhalb der Ausnehmungen 46.

Das Betätigungselement 40 ist von der in Fig. 3 dargestellten zweiten Stellung, in der sich der Rohrschaft 16 in seiner Freigabestellung befindet, in eine dritte Stellung bewegbar, in der das Betätigungselement 40 mit der Ausnehmung 46 vollständig außer Eingriff kommt, wobei der Rohrschaft 16 in der dritten Stellung vom Innenschaft 14 abnehmbar bzw. auf diesem montierbar ist. In dieser dritten Stellung des Betätigungselements 40 liegt die Öffnung 48 des Betätigungselements konzentrisch um den Rohrschaft 16, während die Eingriffsabschnitte 44 und 44' mit den Ausnehmungen 46 am Rohrschaft 16 und insgesamt mit dem Rohrschaft 16 außer Eingriff stehen.

Das Betätigungselement 40 ist weiterhin in einem Führungselement 50 angeordnet, das in Fig. 7 und 8 in Alleinstellung dargestellt ist.

In dem Führungselement 50 ist das Betätigungselement 40 drehfest geführt. Das Betätigungselement 40 ist jedoch relativ zu dem Führungselement 50 axial verschiebbar, wie später noch näher beschrieben wird.

Das Führungselement 50 ist mit einem Bedienelement 52 in Form eines Druckknopfes fest verbunden, das mit dem Daumen der Hand, die den Handgriff 38 hält, bedienbar ist. Zur Befestigung des Führungselements 50 an dem Bedienelement 52 weist das Führungselement an seinem dem Bedienelement 52 zugewandten Ende einen Zapfen 54 mit einem Außengewinde auf, über den das Führungselement 50 in das Bedienelement 52 eingeschraubt ist.

Das Führungselement 50 weist an seinem dem Zapfen 54 zugewandten Ende eine Platte 56 auf, von dessen Außenumfang sich zwei schmale Stege 58 und 60, die einander diametral gegenüberliegen, weg erstrecken. Die beiden Stege 58 und 60 dienen als Drehsicherung und axiale Führung für das Betätigungselement 40, das mit entsprechenden Führungen 62 und 64 ausgestattet ist (Fig. 6). Das Führungselement 50 ist selbst in der Sacklochbohrung 42 drehfest und verschiebbar geführt.

Die Stege 58 und 60 sind an ihrem der Platte 56 gegenüberliegenden Ende mit einem Ring 66 verbunden, der einen lichten Innendurchmesser aufweist, der geringfügig größer als ein Außendurchmesser einer Platte 68 des Betätigungselement 40 ist.

Das Betätigungselement 40 stützt sich mit einer weiteren, der Platte 68 gegenüberliegenden Platte 70 gegen die Platte 56 des Führungselements 50 ab. Bei Niederdrücken des Bedienelements 52 wird somit das Betätigungselement 40 in seiner Bewegungsrichtung quer zum Schaft 12 von dem Führungselement 50, das mit dem Bedienelement 52 fest verbunden ist, mitgenommen.

Das Betätigungselement 40 ist weiterhin gegen eine erste Feder 72 abgestützt. Die erste Feder 72 ist dabei zwischen einem Boden der Sacklochbohrung 42 und einem sich von der Platte 68 des Betätigungselements 40 weg erstreckenden Zapfen 74 angeordnet. Auf den Zapfen 74 ist die erste Feder 72 aufgesteckt.

Des weiteren ist eine zweite Feder 76 vorgesehen, die unterhalb des Rings 66 des Führungselements 50 angeordnet und mit dieser verbunden ist. Die zweite Feder weist einen lichten Innendurchmesser auf, der größer ist als der Außenumfang des Betätigungselements 40, genauer gesagt größer als der Außenumfang der Platte 68 des Betätigungselements 40, so daß die Platte 68 in die zweite Feder 76 eintauchen kann, wie später noch beschrieben wird.

Weiterhin ist die zweite Feder 76 härter als die erste Feder 72.

Die zweite Feder 76, die mit dem Führungselement 50 verbunden ist, ist in der ersten Stellung des Betätigungselements 40 vom Boden der Sacklochbohrung 42 beabstandet und kommt erst in der in Fig. 3 dargestellten zweiten Stellung des Betätigungselements 40 mit dem Boden der Sacklochbohrung 42 in Anlage. Somit ist das Betätigungselement 40 von der in Fig. 2 dargestellten Stellung in die in Fig. 3 dargestellte Stellung gegen die Kraft der ersten Feder 72 allein bewegbar, und muß erst ausgehend von der in Fig. 3 dargestellten zweiten Stellung in die dritte Stellung gemäß Fig. 4 zusätzlich gegen die Kraft der zweiten Feder 76 bewegt werden.

Nachfolgend wird nun die Funktion des Instruments 10 zum Auswechsein des Werkzeugs 18 beschrieben.

In Fig. 2 ist das Instrument 10 in einem Zustand dargestellt, in dem das Werkzeug 18 am Schaft 12 verriegelt angeordnet ist. In diesem Zustand befindet sich das Betätigungselement 40 in seiner ersten Stellung, in die das Betätigungselement 40 vorgespannt ist, und zwar durch die Wirkung der ersten Feder 72.

Ausgehend von der in Fig. 2 dargestellten Stellung kann nun das Bedienelement 52 in Form des Druckknopfes quer zur Längsrichtung des Schafts 12 niedergedrückt werden, bis die in Fig. 3 dargestellte zweite Stellung des Betätigungselements 40 erreicht ist. Diese Stellung des Betätigungselements 40 ist für den Benutzer des Instruments 10 dadurch bemerkbar, daß nunmehr die zweite Feder 76 mit dem Boden der Sacklochbohrung 42 in Anlage kommt, und eine Art Anschlag bildet, der sich durch den höheren Widerstand der nun zusätzlich wirksam werdenden zweiten Feder 76 bemerkbar macht.

Beim Bewegen des Betätigungselements 40 aus der in Fig. 2 dargestellten ersten Stellung in die in Fig. 3 dargestellte zweite Stellung gleiten die Eingriffsabschnitte 44 und 44' des Betätigungselements 40 in den Ausnehmungen 46 des Rohrschafts 16, wodurch sich eine Zwangsführung für den Rohrschaft 16 derart ergibt, daß der Rohrschaft 16 nach proximal in die in Fig. 3 dargestellte zurückgezogene Freigabestellung bewegt wird. In dieser Freigabestellung kommt der sich quer erstreckende Abschnitt 26 des Halters 24 des Werkzeugs 18 mit der Ausnehmung 34 des Rohrschafts 16 außer Eingriff, so daß der Abschnitt 26 des Halters 24 nunmehr in der bajonettartigen Ausnehmung 28 um 90° entgegen dem Uhrzeigersinn gedreht und schließlich aus der Ausnehmung 28 herausgezogen werden kann.

Zum Einsetzen eines neuen Werkzeuges als Austausch für das Werkzeug 18 wird umgekehrt vorgegangen, d.h. während das Betätigungselement 40 in der zweiten Stellung durch Druckausübung auf das Bedienelement 52 in der zweiten Stellung gemäß Fig. 3 gehalten wird, läßt sich ein neues Werkzeug in die bajonettartige Ausnehmung 28 einsetzen und um 90° im Uhrzeigersinn verdrehen, wonach der Druckknopf 52 losgelassen wird und das Betätigungselement 40 mittels seiner Eingriffsabschnitte 44 und 44', die in die Ausnehmungen 46 des Rohrschafts eingreifen, diesen selbsttätig wieder in die in Fig. 2 dargestellte vorgeschobene Verriegelungsstellung nach vorne schiebt. In der zweiten Stellung des Betätigungselements 40 bleiben die Eingriffsabschnitte 44 und 44' mit den Ausnehmungen 46 in Eingriff. Aufgrund der Vorspannung des Betätigungselements 40 in der in Fig. 2 dargestellten ersten Stellung ist der Rohrschaft festgelegt.

Ausgehend von der in Fig. 3 dargestellten zweiten Stellung des Betätigungselements 40 läßt sich das Betätigungselement 40 weiterhin in die in Fig. 4 dargestellte dritte Stellung bewegen, indem das Bedienelement 52 weiter niedergedrückt wird, wobei nun das Betätigungselement 40 sowohl gegen die Kraft der ersten Feder 72 als auch zusätzlich gegen die Kraft der zweiten Feder 76 bewegt werden muß. Das vollständige Niederdrücken des Bedienelements 52 erfolgt dann, wenn der Rohrschaft 16 vom Innenschaft 14 abgenommen werden soll. In der in Fig. 4 dargestellten dritten Stellung kommen die Eingriffsabschnitte 44 und 44' des Betätigungselements 40 nämlich mit den Ausnehmungen 46 am Rohrschaft vollständig außer Eingriff, während die Öffnung 48 des Betätigungselements 40 nunmehr konzentrisch zum Rohrschaft 16 zu liegen kommt, so daß der Rohrschaft aus dem Betätigungselement 40 herausgezogen und somit vollständig vom Innenschaft 14 abgenommen werden kann. Das Werkzeug 18 wurde dazu zuvor gemäß Fig. 3 vom Schaft 12 abgenommen. Nunmehr lassen sich der Rohrschaft 16, der Innenschaft 14 mit dem Handgriff 38 und das Werkzeug 18 separat reinigen.

Zum Montieren des Rohrschafts 16 auf dem Innenschaft 14 bzw. zum Verbinden des Rohrschafts 16 mit dem Handgriff 38 muß jedoch nun nicht mehr das Bedienelement 52, wie in Fig. 4 dargestellt, gegen die Kraft beider Federn 72 und 76 maximal niedergedrückt werden, sondern aufgrund einer weiteren Ausgestaltung des Instruments 10 braucht das Bedienelement 52 dazu nicht betätigt zu werden.

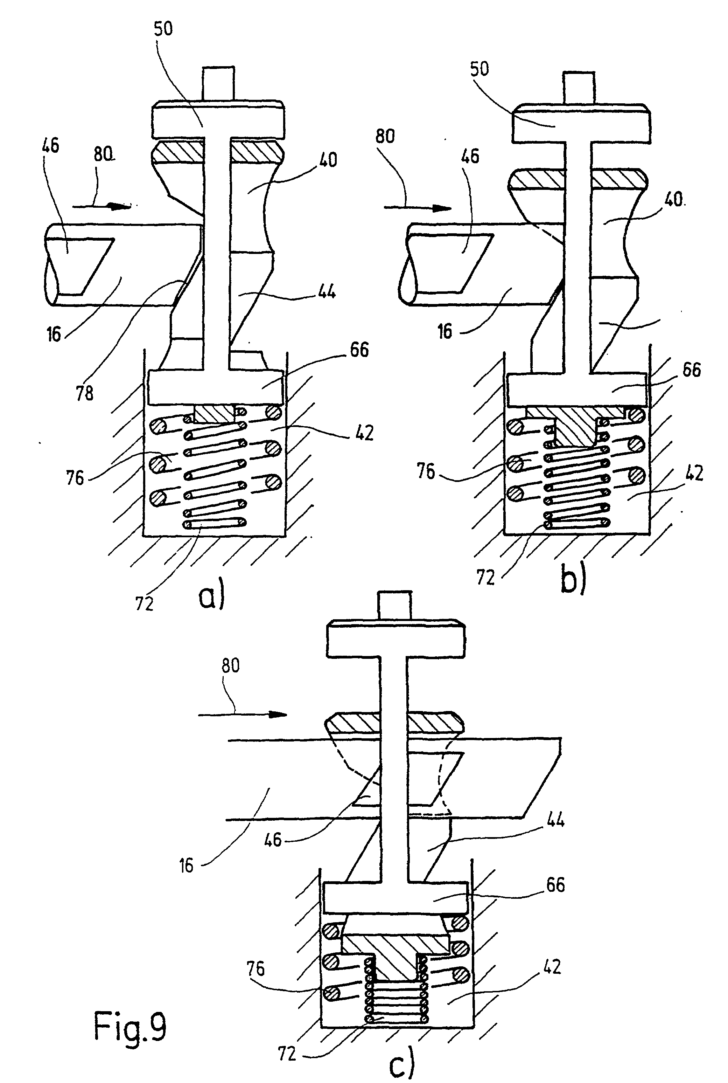

Das Betätigungselement 40 weist nämlich distalseitig eine Anlaufschräge 78 auf, gegen die das proximale Ende des Rohrschafts beim Aufschieben des Rohrschafts 16 auf den Innenschaft 14 anstößt, wie in Fig. 9a) dargestellt ist.

Gemäß Fig. 6 weist das Betätigungselement 40 an seinen beiden Eingriffsabschnitten 44 und 44' distalseitig eine derartige Anlaufschräge 78 und 78' auf.

Durch weiteres Zurückschieben des Rohrschafts 16 gemäß Fig. 9b) wird nun das Betätigungselement 40 ausgehend von seiner ersten Stellung, in die das Betätigungselement 40 durch die erste Feder 72 vorgespannt ist, in die zweite Stellung und über diese hinweg in die dritte Stellung gemäß Fig. 9c) bewegt, ohne daß das Bedienelement 52 und das damit verbundene Führungselement 50 mit bewegt würden. Dies wird dadurch ermöglicht, daß das Betätigungselement 40 relativ zu dem Führungselement 50 beweglich ist, und der Außenumfang des Betätigungselements 40 kleiner ist als der lichte Innendurchmesser des Rings 66 des Führungselements 50 und auch geringer als der lichte Innendurchmesser der zweiten Feder 76. Zum Montieren des Rohrschafts 16 auf dem Innenschaft 14 muß daher das Betätigungselement 40 von seiner ersten Stellung in die dritte Stellung allein gegen die Kraft der ersten Feder 72 und somit gegen eine schwächere Kraft in die dritte Stellung bewegt werden, wobei diese Kraft durch eine Verschiebung des Rohrschafts 16 nach proximal aufgebracht wird.

Ausgehend von Fig. 9c) muß der Rohrschaft 16 nur noch ein kleines Stück weiter nach proximal in Richtung eines Pfeiles 80 verschoben werden, bis die Ausnehmung 46 und die Eingriffsabschnitte 44 und 44' miteinander fluchten, wobei in dieser Stellung die Eingriffsabschnitte 44 und 44' in die Ausnehmungen 46 am Rohrschaft 16 aufgrund der Vorspannung durch die erste Feder 72 selbsttätig einrasten.

Aus dem Vorstehenden ergibt sich, daß das Instrument 10 einen Betätigungsmechanismus mit einer zweistufigen Raste aufweist, deren erste Bewegungsstufe zum Auswechseln des Werkzeugs 18 und deren zweite Stufe zum Abnehmen des Rohrschafts 16 vom Innenschaft 14 dient. Des weiteren ist das Betätigungselement 40 von dem Bedienelement 52 derart entkoppelt, daß zum Montieren des Rohrschafts 16 auf dem Innenschaft 14 das Bedienelement 52 nicht betätigt, sondern der Rohrschaft lediglich aufgeschoben werden muß, wodurch ein selbsttätig wirkender Rastmechanismus geschaffen wird.

## Patentansprüche

1. Medizinisches Instrument, mit einem Schaft (12), und mit einem an einem distalen Ende des Schafts (12) abnehmbar befestigten Werkzeug (18), wobei der Schaft (12) einen Innenschaft (14) und einen den Innenschaft (14) umgebenden und relativ zu diesem längsverschiebbaren Rohrschaft (16) aufweist, wobei das Werkzeug (18) in einer Verriegelungsstellung des Rohrschafts (16) am Schaft (12) verriegelt und in einer Freigabestellung des Rohrschafts (16) vom Schaft (12) abnehmbar ist, **gekennzeichnet durch** ein im wesentlichen quer zur Längsrichtung des Rohrschafts (16) bewegliches Betätigungselement (40), das mit dem Rohrschaft (16) derart in Wirkverbindung steht, daß **durch** eine Bewegung des Betätigungselements (40) von einer ersten Stellung in eine zweite Stellung der Rohrschaft (16) aus der Verriegelungsstellung in die Freigabestellung bewegt wird und umgekehrt.

2. Instrument nach Anspruch 1, **dadurch gekennzeichnet, daß** das Betätigungselement (40) einen Eingriffsabschnitt (44) aufweist, der mit einer Ausnehmung (46) am Rohrschaft (16) relativ zu dieser beweglich in Eingriff steht, wobei der Eingriffsabschnitt (44) und die Ausnehmung (46) eine solche Formgebung aufweisen, daß eine im wesentlichen quer zur Längsrichtung gerichtete Bewegung des Betätigungselements (40) in eine Längsbewegung des Rohrschafts (16) umgesetzt wird.

3. Instrument nach Anspruch 2, **dadurch gekennzeichnet, daß** der Eingriffsabschnitt (44) und die Ausnehmung (46) schräg zur Längsrichtung des Schafts (12) und schräg zur Bewegungsrichtung des Betätigungselements (40) verlaufen.

4. Instrument nach einem der Ansprüche 2 oder 3, **dadurch gekennzeichnet, daß** das Betätigungselement (40) von der zweiten Stellung in eine dritte Stellung bewegbar ist, in der das Betätigungselement (40) mit der Ausnehmung (46) vollständig außer Eingriff kommt, wobei der Rohrschaft (16) in der dritten Stellung vom Innenschaft (14) abnehmbar bzw. auf diesem montierbar ist.

5. Instrument nach einem der Ansprüche 1 bis 4, **dadurch gekennzeichnet, daß** das Betätigungselement (40) in die erste Stellung vorgespannt ist.

6. Instrument nach den Ansprüchen 4 und 5, **dadurch gekennzeichnet, daß** das Betätigungselement (40) von der ersten Stellung in die zweite Stellung gegen eine erste Kraft bewegbar und zum Abnehmen des Rohrschafts (16) vom Innenschaft (14) von der zweiten Stellung in die dritte Stellung gegen eine zweite Kraft bewegbar ist, die größer ist als die erste Kraft.

7. Instrument nach den Ansprüchen 4 und 5 oder nach Anspruch 6, **dadurch gekennzeichnet, daß** das Betätigungselement (40) zur Montage des Rohrschafts (16) auf dem Innenschaft (14) gegen eine erste Kraft allein in die dritte Stellung bewegbar ist.

8. Instrument nach einem der Ansprüche 1 bis 7, **dadurch gekennzeichnet, daß** das Betätigungselement (40) zwei Eingriffsabschnitte (44, 44') aufweist, die an gegenüberliegenden Umfangspostionen des Rohrschafts in entsprechende Ausnehmungen (46) desselben eingreifen.

9. Instrument nach Anspruch 2 und einem der Ansprüche 3 bis 8, **dadurch gekennzeichnet, daß** das Betätigungselement (40) eine sich an den Eingriffsabschnitt (44) oder die Eingriffsabschnitte (44, 44') anschließende Öffnung (48) aufweist, deren lichter Durchmesser größer als der Außendurchmesser des Rohrschafts (16) ist.

10. Instrument nach einem der Ansprüche 1 bis 9, **dadurch gekennzeichnet, daß** das Betätigungselement (40) gegen eine erste Feder (72) abgestützt ist.

11. Instrument nach einem der Ansprüche 1 bis 10, **dadurch gekennzeichnet, daß** das Betätigungselement (40) in einem Führungselement (50) geführt ist, das mit einem Bedienelement (52), beispielsweise in Form eines Druckknopfes, verbunden ist, derart, daß durch Drücken des Bedienelements (52) das Führungselement (50) das Betätigungselement (40) mitnimmt.

12. Instrument nach Anspruch 11, **dadurch gekennzeichnet, daß** das Betätigungselement (40) relativ zu dem Führungselement (50) verschiebbar ist.

13. Instrument nach den Ansprüchen 4 und 10 und nach Anspruch 11 oder 12, **dadurch gekennzeichnet, daß** das Betätigungselement (40) von der zweiten Stellung in die dritte Stellung direkt oder indirekt zusätzlich gegen eine zweite Feder (76) abgestützt bewegbar ist.

14. Instrument nach Anspruch 13, **dadurch gekennzeichnet, daß** sich die zweite Feder (76) gegen das Führungselement (50) abstützt und erst bei der Bewegung des Betätigungselements (40) von der zweiten Stellung in die dritte Stellung zum Abnehmen des Rohrschafts (16) wirksam wird.

15. Instrument nach Anspruch 13 oder 14, **dadurch gekennzeichnet, daß** der lichte Innendurchmesser der zweiten Feder (76) größer ist als ein Außenumfang des Betätigungselements (40), wobei die erste Feder (72) innerhalb der zweiten Feder (76) angeordnet ist.

16. Instrument nach einem der Ansprüche 13 bis 15, **dadurch gekennzeichnet, daß** die zweite Feder (76) härter ist als die erste Feder (72).

17. Instrument nach Anspruch 4 und einem der Ansprüche 3 bis 16, **dadurch gekennzeichnet, daß** am Betätigungselement (40) distalseitig eine Anlaufschräge (78) für das proximale Ende des Rohrschafts (16) ausgebildet ist, so daß das Betätigungselement (40) beim Auflaufen des proximalen Endes auf die Anlaufschräge (78) in die dritte Stellung bewegt wird.

18. Instrument nach einem der Ansprüche 1 bis 17, **dadurch gekennzeichnet, daß** das Betätigungselement (40) an einem Handgriff (38) des Instruments (10) bedienbar angeordnet ist.

19. Instrument nach einem der Ansprüche 1 bis 18, **dadurch gekennzeichnet, daß** das distale Ende des Innenschafts (14) eine bajonettartige Ausnehmung (28) mit einem sich zunächst axial und anschließend teilumfänglich erstreckenden Abschnitt (30, 32) aufweist, und wobei am proximalen Ende des Werkzeugs (18) ein Halter (24) ausgebildet ist, der mit einem sich quer erstreckenden Abschnitt (26) in die bajonettartige Ausnehmung (28) einsetzbar ist, und wobei am Rohrschaft (16) zumindest eine axiale Ausnehmung (34) ausgebildet ist, in die der sich quer erstreckende Abschnitt (26) des Halters (24) in der Verriegelungsstellung des Rohrschafts (16) eingreift.

20. Instrument nach einem der Ansprüche 1 bis 19, **dadurch gekennzeichnet, daß** der Rohrschaft (16) in der Verriegelungsstellung relativ zum Innenschaft (14) vorgeschoben und in der Freigabestellung zurückgezogen ist.

21. Instrument nach einem der Ansprüche 1 bis 20, **dadurch gekennzeichnet, daß** das Werkzeug (18) eine Elektrode (20) zum Koagulieren oder Schneiden von Gewebe ist.

## Claims

1. A medical instrument, comprising a shaft (12), and a tool (18) which is mounted on a distal end of the shaft (12) in such a way as to be removable therefrom, the shaft (12) having an inner shaft (14) and a tubular shaft (16) which surrounds the inner shaft (14) and which is longitudinally displaceable relative thereto, the tool (18) being locked on the shaft (12) when the tubular shaft (16) is in a locking position and being removable from the shaft (12) when the tubular shaft (16) is in a release position, **characterized by** an actuating element (40) which is movable substantially transversely with respect to the longitudinal direction of the tubular shaft (16) and which is connected operatively to the tubular shaft (16) in such a way that the tubular shaft (16) is moved from the locking position to the release position by moving the actuating element (40) from a first position to a second position, and vice versa.

2. The instrument of claim 1, **characterized in that** the actuating element (40) has an engagement portion (44) which engages with a recess (46) on the tubular shaft (16) and is movable relative to said recess, the engagement portion (44) and the recess (46) having such a shape that a movement of the actuating element (40) directed substantially transversely with respect to the longitudinal direction is converted into a longitudinal movement of the tubular shaft (16).

3. The instrument of claim 2, **characterized in that** the engagement portion (44) and the recess (46) extend obliquely with respect to the longitudinal direction of the shaft (12) and obliquely with respect to the direction of movement of the actuating element (40).

4. The instrument of anyone of claims 2 or 3, **characterized in that** the actuating element (40) can be moved from the second position to a third position in which the actuating element (40) completely disengages from the recess (46), and the tubular shaft (16) can be removed from the inner shaft (14) or can be mounted thereon in the third position.

5. The instrument of anyone of claims 1 through 4, **characterized in that** the actuating element (40) is prestressed into the first position.

6. The instrument of claims 4 and 5, **characterized in that** the actuating element (40) can be moved from the first position to the second position counter to a first force and, in order to remove the tubular shaft (16) from the inner shaft (14), can be moved from the second position to the third position counter to a second force which is greater than the first force.

7. The instrument of claims 4 and 5 or of claim 6, **characterized in that**, in order to mount the tubular shaft (16) on the inner shaft (14), the actuating element (40) can be moved into the third position counter to a first force alone.

8. The instrument of anyone of claims 1 through 7, **characterized in that** the actuating element (40) has two engagement portions (44, 44') which engage, at opposite circumferential positions of the tubular shaft, in corresponding recesses (46) thereof.

9. The instrument of claim 2 and anyone of claims 3 through 8, **characterized in that** the actuating element (40) has an opening (48) which adjoins the engagement portion (44) or the engagement portions (44, 44'), a clear diameter of the opening (48) being greater than the external diameter of the tubular shaft (16).

10. The instrument of anyone of claims 1 through 9, **characterized in that** the actuating element (40) is supported against a first spring (72).

11. The instrument of anyone of claims 1 through 10, **characterized in that** the actuating element (40) is guided in a guide element (50) which is connected to an operating element (52), for example in the form of a push button, in such a way that, by pressing the operating element (52), the guide element (50) entrains the actuating element (40).

12. The instrument of claim 11, **characterized in that** the actuating element (40) is displaceable relative to the guide element (50).

13. The instrument of claims 4 and 10 and of claim 11 or 12, **characterized in that** the actuating element (40) can be moved, in addition directly or indirectly supported against a second spring (76), from the second position to the third position.

14. The instrument of claim 13, **characterized in that** the second spring (76) is supported against the guide element (50) and only upon movement of the actuating element (40) from the second position to the third position the second spring (76) is active for removing the tubular shaft (16).

15. The instrument of claim 13 or 14, **characterized in that** the clear internal diameter of the second spring (76) is greater than an external circumference of the actuating element (40), the first spring (72) being arranged inside the second spring (76).

16. The instrument of anyone of claims 13 through 15, **characterized in that** the second spring (76) is harder than the first spring (72).

17. The instrument of claim 4 and anyone of claims 3 through 16, **characterized in that** an abutment bevel (78) for the proximal end of the tubular shaft (16) is formed on the distal side of the actuating element (40), so that the actuating element (40) is moved into the third position when the proximal end runs onto the abutment bevel (78).

18. The instrument of anyone of claims 1 through 17, **characterized in that** the actuating element (40) is arranged to be operated on a hand grip (38) of the instrument (10).

19. The instrument of anyone of claims 1 through 18, **characterized in that** the distal end of the inner shaft (14) has a bayonet-like recess (28) with a portion (30, 32) which initially extends axially and then about part of the circumference, and a holder (24) being arranged at the proximal end of the tool (18), which holder (24) can be inserted with a transversely extending portion (26) into the bayonet-like recess (28), and at least one axial recess (34) being formed on the tubular shaft (16), into which recess (34) the transversely extending portion (26) of the holder (24) engages in the locking position of the tubular shaft (16).

20. The instrument of anyone of claims 1 through 19, **characterized in that** the tubular shaft (16) is pushed forward relative to the inner shaft (14) in the locking position and is pulled back in the release position.

21. The instrument of anyone of claims 1 through 20, **characterized in that** the tool (18) is an electrode (20) for coagulation or cutting of tissue.

## Revendications

1. Instrument médical avec une tige (12) et avec un outil (18) fixé de manière séparable à une extrémité distale de la tige (12), la tige (12) comportant une tige intérieure (14) et une tige tubulaire (16) entourant la tige intérieure (14) et pouvant coulisser dans le sens de la longueur par rapport à celle-ci, l'outil (18) étant verrouillé sur la tige (12) dans une position de verrouillage de la tige tubulaire (16), et pouvant être séparé de la tige (12) dans une position de dégagement de la tige tubulaire (16), **caractérisé par** un élément d'actionnement (40) déplaçable sensiblement perpendiculairement à la direction longitudinale de la tige tubulaire (16) et qui est en liaison fonctionnelle avec la tige tubulaire (16) de manière que, par un déplacement de l'élément d'actionnement (40) d'une première position dans une deuxième position, la tige tubulaire (16) soit déplacée de la position de verrouillage dans la position de dégagement et inversement.

2. Instrument selon la revendication 1, **caractérisé en ce que** l'élément d'actionnement (4) comporte une portion d'engagement (44) qui, par un évidement (46) de la tige tubulaire (16), est en engagement avec possibilité de déplacement par rapport à celle-ci, la portion d'engagement (44) et l'évidement (46) présentant une forme telle qu'un mouvement de l'élément d'actionnement (40), dirigé sensiblement perpendiculairement à la direction longitudinale, soit transformé en un mouvement longitudinal de la tige tubulaire (16).

3. Instrument selon la revendication 2, **caractérisé en ce que** la portion d'engagement (44) et l'évidement (46) s'étendent obliquement par rapport à la direction longitudinale de la tige (12) et obliquement par rapport à la direction de déplacement de l'élément d'actionnement (40).

4. Instrument selon l'une des revendications 2 ou 3, **caractérisé en ce que** l'élément d'actionnement (40) est déplaçable de la deuxième position dans une troisième position, dans laquelle l'élément d'actionnement (40) est totalement dégagé de l'évidement (46), la tige tubulaire (16) dans sa troisième position pouvant être séparée de la tige intérieure (14) ou montée sur celle-ci.

5. Instrument selon l'une des revendications 1 à 4, **caractérisé en ce que** l'élément d'actionnement (40) est précontraint dans la première position.

6. Instrument selon les revendications 4 et 5, **caractérisé en ce que** l'élément d'actionnement (40) est déplaçable de la première position dans la deuxième position à l'encontre d'une première force et, pour séparer la tige tubulaire (16) de la tige intérieure (14), il est déplaçable de la deuxième position dans la troisième position à l'encontre d'une deuxième force qui est supérieure à la première force.

7. Instrument selon les revendications 4 et 5 ou selon la revendication 6, **caractérisé en ce que** pour le montage de la tige tubulaire (16) sur la tige intérieure (14), l'élément d'actionnement (40) est déplaçable à l'encontre d'une première force seulement dans la troisième position.

8. Instrument selon l'une des revendications 1 à 7, **caractérisé en ce que** l'élément d'actionnement (40) comporte deux portions d'engagement (44, 44') qui s'engagent, en des positions périphériques opposées de la tige tubulaire, dans des évidements (46) correspondants de celle-ci.

9. Instrument selon la revendication 2 et l'une des revendications 3 à 8, **caractérisé en ce que** l'élément d'actionnement (40) présente une ouverture (48) faisant suite à la portion d'engagement (40) ou aux portions d'engagement (44, 44'), dont le diamètre intérieur est supérieur au diamètre extérieur de la tige tubulaire (16).

10. Instrument selon l'une des revendications 1 à 9, **caractérisé en ce que** l'élément d'actionnement (40) est appuyé contre un premier ressort (72).

11. Instrument selon l'une des revendications 1 à 10, **caractérisé en ce que** l'élément d'actionnement (40) est guidé dans un élément de guidage (50) qui est relié à un élément de commande (52), par exemple sous la forme d'un bouton-poussoir, de manière que lorsqu'on enfonce l'élément de commande (52), l'élément de guidage (50) entraîne l'élément d'actionnement (40).

12. Instrument selon la revendication 11, **caractérisé en ce que** l'élément d'actionnement (40) peut coulisser par rapport à l'élément de guidage (50).

13. Instrument selon les revendications 4 et 10 et selon la revendication 11 ou 12, **caractérisé en ce que** l'élément d'actionnement (40) est déplaçable de la deuxième position dans la troisième position, directement ou indirectement appuyé en outre contre un deuxième ressort (76).

14. Instrument selon la revendication 13, **caractérisé en ce que** le deuxième ressort (76) est appuyé contre l'élément de guidage (50) et n'opère pour la séparation de la tige tubulaire (16) que pendant le déplacement de l'élément d'actionnement (40) de la deuxième position à la troisième position.

15. Instrument selon la revendication 13 ou 14, **caractérisé en ce que** le diamètre intérieur libre du deuxième ressort (76) est supérieur à un périmètre extérieur de l'élément d'actionnement (40), le premier ressort (72) étant disposé à l'intérieur du deuxième ressort (76).

16. Instrument selon l'une des revendications 13 à 15, **caractérisé en ce que** le deuxième ressort (76) est plus raide que le premier ressort (72).

17. Instrument selon la revendication 4 et l'une des revendications 3 à 16, **caractérisé en ce que** sur l'élément d'actionnement (40) est réalisée côté distal une surface oblique de butée (78) pour l'extrémité proximale de la tige tubulaire (16), de manière que l'élément d'actionnement (40) soit déplacé dans la troisième position, lorsque l'extrémité proximale vient heurter la surface oblique de butée (78).

18. Instrument selon l'une des revendications 1 à 17, **caractérisé en ce que** l'élément d'actionnement (40) est disposé, de manière commandable, sur une poignée (38) de l'instrument (10).

19. Instrument selon l'une des revendications 1 à 18, **caractérisé en ce que** l'extrémité distale de la tige intérieure (14) présente un évidement (28) de type baïonnette avec une portion (30, 32) s'étendant d'abord axialement puis partiellement périphériquement, à l'extrémité proximale de l'outil (18) étant réalisé un support (24) qui, par une portion (26) s'étendant transversalement, peut être inséré dans l'évidement (28) de type baïonnette, et sur la tige tubulaire (16) étant ménagé au moins un évidement axial (34) dans lequel la portion (26) s'étendant transversalement du support (24) s'engage, dans la position de verrouillage de la tige tubulaire (16).

20. Instrument selon l'une des revendications 1 à 19, **caractérisé en ce que** la tige tubulaire (16) est avancée par rapport à la tige intérieure (14) dans la position de verrouillage, et est reculée dans la position de dégagement.

21. Instrument selon l'une des revendications 1 à 20, **caractérisé en ce que** l'outil (18) est une électrode (20) pour coaguler ou couper un tissu,
